# EUROPÄISCHE PATENTSCHRIFT

(11) **EP 2 804 555 B1**
(45) Veröffentlichungstag und Bekanntmachung des Hinweises auf die Patenterteilung: **21.11.2018**
(21) Anmeldenummer: 13702192.9
(22) Anmeldetag: 10.01.2013
(51) Int. Cl.: A61B 18/14, A61B 18/00

(54) **TFT-KOAGULATIONSSICHERUNG**
TFT COAGULATION SAFEGUARD
PROTECTION D'UN DISPOSITIF DE COAGULATION TFT

(30) Priorität: 19.01.2012 DE 102012100425
(43) Veröffentlichungstag der Anmeldung: 26.11.2014
(73) Patentinhaber: Aesculap AG, 78532 Tuttlingen (DE)
(72) Erfinder: WEISSHAUPT, Dieter, 78194 Immendingen (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2013/050403
(87) Internationale Veröffentlichungsnummer: WO 2013/107685

(56) Entgegenhaltungen:
- WO-A1-99/12488
- WO-A1-2011/006937
- DE-A1-102010 020 664
- DE-U1-202010 013 150

## Beschreibung

Die vorliegende Erfindung betrifft eine TFT-Koagulationssicherung und insbesondere ein TFT-Insturment mit einer schwenk- bzw. abklappbare HF-Elektrode für End-zu-End-Anastomose mit integrierter HF-Energie-Unterbrechung gemäß dem Oberbegriff des Anspruchs 1.

Unter einer End-zu-End Anastomose versteht man grundsätzlich eine (künstliche) Anastomose, bei der zwei Hohlorganabschnitte (z.B. Darmteile) an ihren eröffneten Enden wieder aneinander genäht werden, so dass ein fluiddichter sowie kontinuierlicher Verlauf entsteht. Wichtig hierbei ist es, dass die umlaufende Naht eine vollständig fluiddichte sowie flexible und reißfeste Verbindung der zwei zu verbindenden Hohlorganabschnitte ergibt, die derart gestaltet ist, dass sich an der Hohlorganinnenseite eine möglichst glatte Oberfläche für die Gewährleistung einer störungsfreien Fluidströmung innerhalb des Hohlorgans ausbildet.

Aus dem Stand der Technik sind Klammergeräte in Form von Ein-Weg-Instrumenten bekannt, wie es beispielsweise in der DE 43 27 233 A1 offenbart ist. Demzufolge hat ein Klammergerät dieser Gattung einen Endoskopschaft, an dessen proximalem Endabschnitt ein Handstück zum Halten des Geräts sowie zum Betätigen eines Klammermechanismus angeordnet ist. Der Klammermechanismus besteht aus einem distal am Endsokopschaft fixierten Klammermagazin etwa in Form einer zylindrischen Patronenhülse, in der eine Anzahl vom auf einem Kreis umlaufend angeordneten Klammern federvorgespannt gelagert sind sowie einem Klammerkopf oder Klammerkappe die pilz- oder schirmartig am axial distalen Ende der Patronenhülse platziert und mittels eines daran fixierten Stils/Stifts axial beweglich in der Patronenhülse geführt ist. Der Klammerkopf ist somit in Axialrichtung des Klammergeräts von der Patronenhülse kontinuierlich beabstandbar, wodurch sich zwischen dem Klammerkopf und der Patronenhülse ein einstellbarer, umlaufender (ringförmiger) Axialspalt ergibt, in den die beiden zu verbindenden Enden des Hohlorgans eingeklemmt werden können. Der Klammerkopf (oder Klammerkappe) sowie die Patronenhülse sind über geeignete Übertragungsmittel innerhalb des Endoskopschafts vom Handstück aus betätigbar.

Das Problem derartiger Klammergeräte besteht grundsätzlich darin, eine fluiddichte sowie flexible und reißfeste Naht herzustellen. Zu diesem Zweck werden bei manchen Klammergeräten zwei in Radialrichtung der Patronenhülse beabstandete Klammerreihen vorgesehen, deren Klammern sich in ihren Eingriffsbereichen überlappen. Zwar wird hierdurch der Dichtigkeitsgrad sowie die Reißfestigkeit verbessert, indessen entstehen zwangsläufig radial nach innen vorragende Gewebelappen oder -falten, wodurch ein Fluidstrom innerhalb des Hohlorgans behindert werden kann. Aus diesem Grund werden Klammergeräte dieser Gattung neuerdings durch medizinische TFT-Instrumente vorzugsweise mit bipolarem Elektrodenaufbau ersetzt bzw. ergänzt.

Auch in diesem Fall hat ein solches TFT-Instrument einen Endoskopschaft mit einem proximalen Handstück sowie einem Endoskopkopf, der vorzugsweise mechanisch sowie elektrisch mit dem Handstück über geeignete Übertragungsmittel innerhalb des Endoskopschafts verbunden ist. Der Endoskopkopf besteht im Wesentlichen aus einer Lagerhülse, die an den distalen Endabschnitt des Endoskopschafts axial sich anschließend fest angesetzt ist und an deren distaler Stirnkante eine erste umlaufende Elektrode (Elektrodenring) montiert ist sowie aus einem relativ bewegbaren Elektrodenschirm (Elektrodenkappe). Der Elektrodenschirm hat hierfür einen axialen Stift, der in der Lagerhülse axialverschiebbar geführt und mit einem mechanischen Übertragungsmittel innerhalb des Endoskopschafts für dessen Betätigung über das Handstück gekoppelt/koppelbar ist sowie einen umlaufenden Elektrodenkranz, der am distalen Ende des Stifts an diesem angeordnet ist. An der der Lagerhülse zugewandten Stirnseite des Elektrodenkranzes trägt dieser eine zweite umlaufende Elektrode (Elektrodenring), die mit der ersten Elektrode zusammenwirkt.

In Übereinstimmung mit den an sich bekannten Klammergeräten kann auch beim TFT-Instrument dieses Aufbaus durch axiales Bewegen des Elektrodenschirms mittels des Handstücks ein Axialspalt zwischen dem Elektrodenkranz und der Lagerhülse eingestellt werden, um so zu verbindendes Gewebe dazwischen einzuspannen und durch HF-Strombeaufschlagung der Elektroden zu koagulieren (zu verschweißen bzw. zu versiegeln). Durch dieses Verfahren entsteht eine flexible sowie fluiddichte Gewebenaht, ohne dass große Gewebefalten in das Innere des Hohlorgans vorstehen.

Indessen besteht bei derartigen TFT-Geräten das Problem, dass zumindest der Schirm beim Herausziehen des Geräts aus dem Hohlorgan die Nahtstelle passieren muss, ohne diese nachhaltig zu verletzen. Dabei besteht immer das Risiko, dass die HF-Stromzufuhr unbeabsichtigt aktiviert wird, wodurch u.U. Gewebe insbesondere im Nahtbereich geschädigt werden kann.

Angesichts dessen besteht eine Aufgabe der vorliegenden Erfindung darin, ein gattungsgemäßes medizinisches Instrument der bipolaren Bauart, nachfolgend als TFT-Gerät bezeichnet, so weiter zu entwickeln, dass die Gefahr der unbeabsichtigten Gewebeschädigung durch HF-Einwirkung insbesondere beim Entnehmen des Geräts aus einem Organ (vorzugsweise Hohlorgan) reduziert wird.

Diese Aufgabe wird durch ein TFT-Gerät mit den Merkmalen des Anspruchs 1 gelöst. Vorteilhafte Ausgestaltungen der Erfindung sind Gegenstand der Unteransprüche.

Der Grundgedanke der Erfindung besteht prinzipiell darin, die HF-Stromzufuhr zu zumindest einer der Elektroden eines erfindungsgemäßen bipolaren TFT-Geräts vorzugsweise der Ein-Weg-Bauart über einen distalen Sicherungsschaltermechanismus (zusätzlich zu einem Hauptschalter im Bereich des Handstücks) zu führen, der an/bei zumindest einem der Elektrodenträger angeordnet (integriert) ist und der bei Überführen des TFT-Geräts bzw. des einen Elektrodenträgers in Funktionsstellung / Funktionssituation (Situation, in der die Elektroden bestrombar sein sollen) selbsttätig (d.h. automatisch durch die Bewegung des zumindest einen Elektrodenträgers) in Ein-Position sowie bei Überführen des Elektrodenträgers in Außerfunktionsstellung/Außerfunktionssituation (Situation, in der die Elektroden nicht mehr bestrombar sein sollen) in Aus-Position geschaltet/gebracht wird, wodurch eine HF-Stromzufuhr zwangsläufig (unabhängig von der Hauptschalterbetätigung) unterbrochen ist. Durch diese Trennung der HF-Zuleitung am Elektrodenträger kann eine ungewollte Gewebeschädigung nicht mehr erfolgen, auch dann nicht, wenn versehentlich eine HF-Aktivierung etwa durch entsprechendes Betätigen am Handgriff ausgelöst wird.

Im konkreten Fall ist das TFT-Gerät ein End-zu-End Anastomose HF-Instrument mit einem Endoskopschaft, an dessen distalem Endstück eine im Wesentlichen ringförmige erste Elektrode (oder Elektrodenreihe) stirnseitig platziert ist und einem Elektrodenschirm oder -Elektrodenkappe, die an einem (röhrchenförmigen) Stift oder Stil endseitig montiert ist, der in dem distalen Endstück (Lagerhülse) des Endoskopschafts axialverschiebbar gelagert ist. Die Elektrodenkappe bildet dabei eine Art kranzförmiger Elektrodenträger, der an einer dem Endstück zugewandten Seite eine zweite, im Wesentlichen ringförmige Elektrode (oder Elektrodenreihe) trägt, welche mit der ersten Elektrode zusammenwirkt.

Erfindungsgemäß ist der kranzförmige Elektrodenträger, klapp- oder schwenkbar am (röhrchenförmigen) Stift (scharnierartig) zentrisch gelagert. Die Verschwenkung oder Abklappung des kranzförmigen Elektrodenträgers bezüglich des Stifts erfolgt mittels eines im/am Stift gelagerten Betätigungsstabs (Nadel), der über zumindest einen Hebel mit dem kranzförmigen Elektrodenträger gelenkig verbunden ist, wodurch eine Axialbewegung des Betätigungsstabs längs des Stifts in eine Schwenk- oder Klappbewegung des kranzförmigen Elektrodenträgers um den Stift transferiert wird. Der Betätigungsstab ist dabei elektrisch mit der vom Elektrodenträger gehaltenen Elektrode (Elektrodenreihe) verbunden und stellt somit einen Abschnitt der HF-Zuleitung dar. Am (röhrchenförmigen) Stift vorzugsweise aus elektrisch nicht leitendem Material ist ein (einzelner) Berührkontakt, beispielsweise in Form eines Schleifkontakts, fixiert (angeordnet/ausgebildet), der lediglich bei/ab einer vorbestimmten Axialposition des Betätigungsstabs bezüglich des Stifts, in welcher der kranzförmige Elektrodenträger im Wesentlichen in Funktionsstellung ausgeklappt bzw. ausgeschwenkt ist, mit dem Betätigungsstab oder einem daran vorgesehenen Kontakt in elektrischen Kontaktschluss kommt und somit die HF-Zuleitung herstellt. Demzufolge wird die HF-Zuleitung außerhalb der vorbestimmten Axialposition des Betätigungsstabs bezüglich des Stifts getrennt. Der Betätigungsstab sowie die Kontakt auf Seiten des Stifts bilden somit den erfindungsgemäßen Sicherheitsschaltmechanismus.

Zusammenfassend ist demnach der Sicherheitsschaltmechanismus an eine mechanische Funktion des HF-Instruments gekoppelt, mit welcher zumindest zwei HF-Elektroden relativ zueinander bewegbar sind, um in eine Funktions- und/oder Außerfunktionsstellung gebracht zu werden.

Die Erfindung wird nachstehend anhand eines bevorzugten Ausführungsbeispiels unter Bezugnahme auf die begleitenden Figuren näher erläutert.
Fig. 1 zeigt ein medizinisches bipolares TFT-Instrument vorzugsweise der End-zu-End-Anastomose-Bauart mit einer Anzahl von alternativen axialverschiebbaren Elektrodenkappen gemäß der Erfindung und
Fig. 2a-2d zeigen eine Variante einer axialverschiebbaren Elektrodenkappe gemäß der Erfindung in Funktions- und Außerfunktionsstellung im Detail.

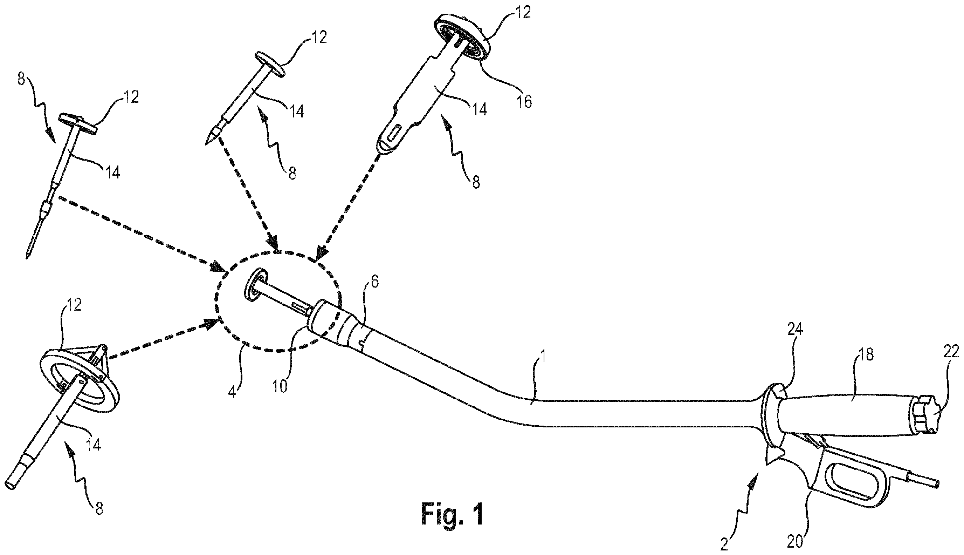

In der Fig. 1 ist ein bipolares medizinisches Instrument vorzugsweise der End-zu-End Anastomose Bauart grundsätzlich dargestellt.

Das beispielhaft vorliegende Instrument der End-zu-End Anastomose-Bauart gemäß der Fig. 1 hat einen vorzugsweise biegestarren (d.h. biegestarrer als das ihn umgebende Organgewebe und somit der Hohlorganform sich nicht anpassend) Endoskopschaft 1, an dessen proximalem Ende ein Handstück 2 angeordnet ist. Am distalen Ende des Endsokopschafts 1 ist ein Endoskopkopf 4 vorgesehen im Wesentlichen bestehend aus einer am Endoskopschaft 1 axial fixierten oder daran ausgebildeten Lagerhülse 6 und einer in der Lagerhülse 6 axial beweglich geführten Elektrodenkappe (Elektrodenschirm) 8. An der distalen Stirnseite der Lagerhülse 6 ist ein erster Elektrodenring (oder umlaufende Elektrodenreihe) 10 eingesetzt, der über eine im Endoskopschaft 1 verlegte elektrische Zuleitung (nicht dargestellt) mit einem proximalen Hauptschalter (ebenfalls nicht gezeigt) im Bereich des Handstücks 2 verbunden ist.

Die Elektrodenkappe 8 besteht aus einem kranzförmigen Elektrodenhalter 12, der zentral mit einem Stift 14 gekoppelt ist, derart, dass der kranzförmige Elektrodenhalter 12 entweder komplett vom Stift 14 abgetrennt, oder gegenüber dem Stift 14 verschwenkt bzw. eingeklappt werden kann, wie dies nachfolgend noch näher beschrieben wird. Der Stift 14 ist wiederum axial verschiebbar in der distalen Endoskop-Lagerhülse 6 eingesetzt oder einsetzbar. Zur Betätigung des Schwenk-/Klapp-/Abtrennmechanismus sowie zur Axialverschiebung des Stifts 14 innerhalb der distalen Lagerhülse 6 sind im Endoskopschaft 1 eine Anzahl von Schub-/Zugstangen oder Zügen verlegt (nicht dargestellt), die distal an die Elektrodenkappe 8 und proximal an das Handstück 2 bzw. an vorbestimmte Handhaben des Handstücks 2 gekoppelt sind. Schließlich ist am kranzförmigen Elektrodenhalter 12 an dessen der Lagerhülse 6 zugewandten Stirnseite ein zweiter Elektrodenring (oder umlaufende Elektrodenreihe) 16 montiert, der über eine vorzugsweise separate Zuleitung innerhalb des Endoskopschafts 1 ebenfalls mit dem Hauptschalter elektrisch verbunden/verbindbar ist.

Das Handstück 2 besteht gemäß der Fig. 1 aus einem ergonomisch geformten Handgriff 18 sowie einem relativ hierzu bewegbaren/schwenkbaren Hebel oder Trigger (erste Handhabe) 20, über den der integrierte (nicht weiter gezeigte) Hauptschalter zur HF-Stromzufuhr zu den Elektroden 10, 16 betätigbar ist. Des Weiteren sind am Handstück 2 eine Anzahl von Stellrädern oder Knöpfen (zweite und dritte Handhabe) 22, 24 zur Aktivierung der Schub-/Zugstangen für eine axiale Verschiebung der Elektrodenkappe 8 bezüglich der Lagerhülse 6 sowie zur Betätigung des Schwenk-/Klappmechanismus des kranzförmigen Elektrodenhalters 12 angebracht.

Der generelle konstruktive Aufbau des erfindungsgemäßen TFT-Geräts zur mechanischen Kopplung der Elektrodenkappe an den Endoskopschaft sowie zur elektrischen Verbindung der Elektroden mit dem Hauptschalter sind aus dem Stand der Technik hinlänglich bekannt, sodass an dieser Stelle auf eine weitere detaillierte Beschreibung verzichtet werden kann. In sofern werden nachstehend im Wesentlichen die einzelnen Varianten der erfindungsgemäßen Elektrodenkappen anhand der Fig. 1 bis 3 näher beschrieben.

Wie ebenfalls aus der Fig. 1 zu entnehmen ist, kann das erfindungsgemäße TFT-Gerät vorliegend in Form des End-zu-End-Anastomose-Instruments mit unterschiedlich gestalteten Elektrodenkappen 8 bestückt werden. Grundsätzlich hat jede Elektrodenkappe 8 den vorstehend bereits genannten kranzförmigen Elektrodenhalter 12 sowie den lotrecht hierzu sich erstreckenden Stift 14, der in die Lagerhülse 6 einführbar und mit einer Betätigungsvorrichtung zumindest zur Axialverschiebung der Elektrodenkappe 8 koppelbar ist. Je nach Ausführungsvariante ist der Elektrodenhalter 12 bezüglich des Stifts 14 (scharnierartig) schwenkbar gelagert oder der Elektrodenhalter 12 ist mehrgeteilt, wobei jedes Teil bezüglich des Stifts 14 umgeklappt werden kann.

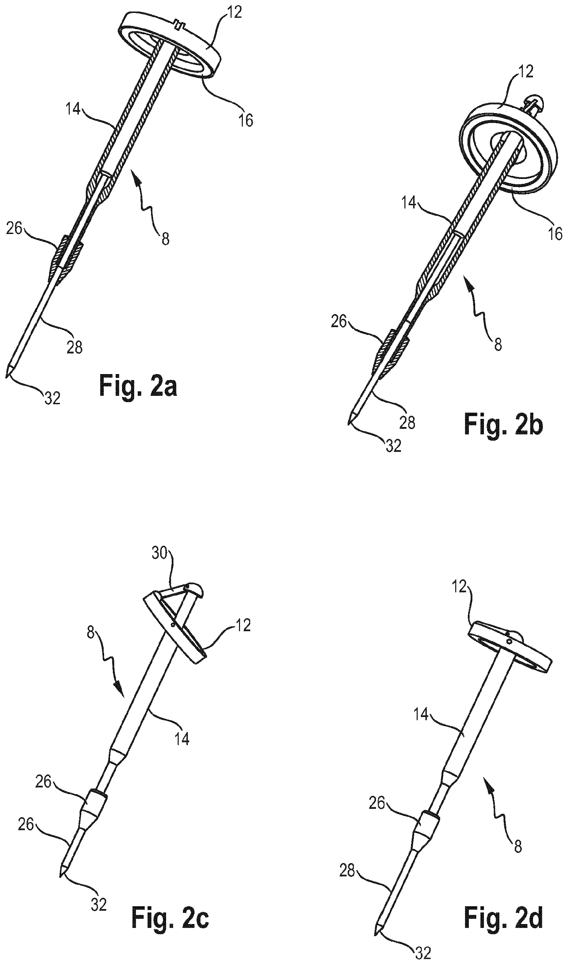

Die Fig. 2a bis 2d zeigen nunmehr eine Elektrodenkappe 8 dieser Erfindung gemäß einem ersten bevorzugten Ausführungsbeispiel in Funktions- sowie in Außerfunktionsstellung/-situation.

In der Fig. 2a ist die Elektrodenkappe 8 im Teillängsschnitt sowie in Funktionsstellung dargestellt. Demnach besteht der Stift 14 aus einem Röhrchen aus vorzugsweise nicht leitendem oder elektrisch isolierten Material, an dessen distalem Ende der kranzförmige Elektrodenhalter 12 zentrisch anscharniert ist, derart, dass dieser bezüglich des röhrchenförmigen Stifts 14 verschwenken/verkippen kann, wie dies in den Fig. 2b und 2c gezeigt ist. An einem proximalen Endabschnitt des röhrchenförmigen Stifts 14 ist ein äußerer Kontaktring 26 aus elektrisch leitendem Material fixiert, der vorzugsweise in das Innere des Röhrchens 14 bereichsweise vorragt. In dem Röhrchen 14 steckt axialverschiebbar ein Betätigungsstab oder Nadel 28, welche am distalen Ende aus dem Röhrchen 14 vorragt und an deren vorragenden, distalen Ende ein Gelenkhebel 30 anscharniert ist, der wiederum mit dem kranzförmigen Elektrodenträger 12 randseitig sowie gelenkig verbunden ist (siehe insbesondere Fig. 2c). Wird folglich der Betätigungsstab 28 axial im Röhrchen 14 verschoben, überträgt sich diese Bewegung auf den Elektrodenträger 12, wobei sich dieser bezüglich des Röhrchens 14 verkippt.

Der Betätigungsstab 28 ist dabei aus einem elektrisch leitenden Material gefertigt (oder hat eine elektrische Leitung) und ist elektrisch mit dem Elektrodenring (umlaufende Elektrodenreihe) 16 am Elektrodenhalter 12 verbunden. Der Betätigungsstab 28 bzw. die daran montierte Leitung ist so geformt, dass er/sie bei einer bestimmten Axialposition bezüglich des Röhrchens 14, in welcher der Elektrodenträger 12 im Wesentlichen seine Funktionsstellung einnimmt (siehe Fig. 2a und 2d), mit dem äußeren Kontaktring 26 in Anlage kommt und somit einen Kontaktschluss mit der im Endoskopschaft 1 verlegten elektrischen Leitung herstellt. Schließlich ragt der Betätigungsstab 28 auch am proximalen Ende aus der Röhrchen 14 vor und bildet an seinem proximalen Ende einen Eingriffsabschnitt 32, der mit einem entsprechenden Betätigungsmechanismus im Endoskopschaft 1 koppelbar ist.

Wird demnach der Betätigungsstab 28 aus der Axialposition (entspricht der Funktionsstellung) gemäß der Fig. 2a oder 2b, in welcher der Elektrodenträger 12 im Wesentlichen parallel zur distalen Stirnseite der Lagerhülse 6 ausgerichtet ist, in eine Axialposition (entspricht der Außerfunktionsstellung) gemäß der Fig. 2b oder 2c verschoben, in welcher der Elektrodenträger 12 einen Winkel > O° bezüglich der distalen Stirnseite der Lagerhülse 6 einnimmt, wird gleichzeitig der elektrischen Kontakt zwischen dem äußeren Kontaktring 26 und dem Betätigungsstab 28 (automatisch) getrennt, sodass die kappenseitige Elektrode 16 nicht mehr mit einem HF-Strom beaufschlagbar ist. Selbst wenn in dieser Stellung der Hauptschalter am Hand-/Griffstück 2 (unbeabsichtigt) betätigt werden sollte, kann die kappenseitige Elektrode 16 nicht aktiviert werden. Eine Beschädigung des umliegenden Gewebes kann so sicher vermieden werden.

An dieser Stelle sei darauf hingewiesen, dass die Schwenklagerung des kranzförmigen Elektrodenhalters 12 auch durch eine gleichwirkende hierzu äquivalente Klapplagerung ersetzt werden kann, wie sie schematisch in der Fig. 1 bei einer dort gezeigten Variante angedeutet ist. In diesem Fall kann der Elektrodenhalter 12 zweigeteilt sein, wobei die beiden Teile scharnierartig miteinander gekoppelt sind. Jedes Teil ist dabei über einen eigenen Gelenkhebel mit dem Betätigungsstab 28 wirkverbunden, sodass die beiden Teile bei Axialverschieben des Betätigungsstabs 28 ähnlich zu einem Schirm aufgeklappt/aufgespannt werden können.

Zusammenfassend wird ein medizinisches TFT-Instrument der bipolaren Bauart offenbart mit zumindest zwei von relativ zueinander bewegbaren Elektrodenträgern 6, 12 gehaltenen Elektroden 10, 16, die über jeweils eine Zuleitung mit HF-Strom beaufschlagbar sind. Erfindungsgemäß ist ein im Bereich der Elektrodenträger 6, 12 angeordneter, sowie in zumindest eine der Zuleitungen zwischengeschalteter Sicherungsschaltermechanismus zusätzlich vorgesehen, der bei Überführen zumindest eines der Elektrodenträger 12 in Außerfunktionsposition/-situation in eine Aus- oder Trennstellung gebracht wird, wodurch eine HF-Stromzufuhr zu der dieser Zuleitung zugeordneten Elektrode unterbrochen ist.

## Patentansprüche

1. Medizinisches Instrument der bipolaren Bauart mit zumindest zwei von relativ zueinander bewegbaren Elektrodenträgern (6, 12) gehaltenen Elektroden (10, 16), die über jeweils eine Zuleitung mit HF-Strom beaufschlagbar sind, **gekennzeichnet durch** einen im Bereich zumindest eines der Elektrodenträger (6, 12) angeordneten, sowie in zumindest eine der Zuleitungen zwischengeschalteten Sicherungsschaltermechanismus (26, 28, 34), der erst bei Überführen eines ersten, kranzförmigen Elektrodenträgers (12) in Außerfunktionsposition in eine Aus- oder Trennstellung gebracht wird, wodurch eine HF-Stromzufuhr zu der der Zuleitung zugeordneten Elektrode (16) unterbrochen ist, mit einem Endoskopschaft (1), dessen distales Endstück den anderen Elektrodenträger (6) bildet, an welchem die im Wesentlichen ringförmige erste Elektrode (10) stirnseitig platziert ist und einer Elektrodenkappe (8) mit einem Stift (14), der in dem distalen Endstück (6) des Endoskopschafts (1) axial verschiebbar gelagert ist, wobei die Elektrodenkappe (8) an einer dem Endstück (6) zugewandten Seite eine zweite, im Wesentlichen ringförmige Elektrode (16) trägt, welche mit der ersten Elektrode (10) zusammenwirkt, wobei die Elektrodenkappe (8) aus dem kranzförmigen Elektrodenträger (12) besteht, der scharnierartig am nunmehr röhrchenförmigen Stift (14) zentrisch gelagert ist, um in eine Funktionsstellung im Wesentlichen parallel zur Stirnseite des distalen Schaftendstücks (6) und eine Außerfunktionsstellung in einem Winkel größer 0° zur Stirnseite des distalen Schaftendstücks (6) geschwenkt zu werden, wobei der Sicherungsschaltermechanismus (26, 28 34) mechanisch mit dem Stift (14) gekoppelt ist, derart, dass bei einer Stellung des kranzförmigen Elektrodenträgers (12) außerhalb seiner Funktionsstellung die zumindest eine Zuleitung zu der vom kranzförmigen Elektrodenträger (12) gehaltenen Elektrode (16) unterbrochen ist, wobei die Verschwenkung des kranzförmigen Elektrodenträgers (12) bezüglich des Stifts (14) mittels eines im Stift (14) axialbeweglich gelagerten Betätigungsstabs (28) erfolgt, der über einen Gelenkhebel (30) mit dem kranzförmigen Elektrodenträger (12) gekoppelt ist, um eine Axialbewegung des Betätigungsstabs (28) längs des Stifts (14) in eine Schwenkbewegung des kranzförmigen Elektrodenträgers (12) um den Stift (14) zu transferieren und wobei der Betätigungsstab (28) elektrisch mit der vom kranzförmigen Elektrodenträger (12) gehaltenen Elektrode (16) verbunden ist und somit einen Abschnitt der HF-Zuleitung darstellt, wobei am röhrchenförmigen Stift (14) ein Berührkontakt (26) angeordnet ist, der bei einer vorbestimmten Axialposition des Betätigungsstabs (28) bezüglich des Stifts (14), in welcher der kranzförmige Elektrodenträger (12) in Funktionsposition ausgeklappt ist, mit dem Betätigungsstab (28) oder einem daran vorgesehenen Kontakt in elektrischen Kontaktschluss kommt.

2. Medizinisches Instrument nach Anspruch 1, **dadurch gekennzeichnet, dass** es ein End-zu-End-Anastomose-HF-Instrument vorzugsweise der Ein-Weg-Bauart ist.

## Claims

1. A medical instrument of bipolar design comprising at least two electrodes (10, 16) being held by electrode carriers (6, 12) movable relative to each other which can be charged with HF current via a respective feed line,
**characterized by** a safety switch mechanism (26, 28, 34) arranged in the area of at least one of the electrode carriers (6, 12) and interconnected in at least one of the feed lines, wherein the safety switch mechanism (26, 28, 34) is brought into an OFF or disconnect position only upon transferring a first, rim-shaped electrode carrier (12) into the out-of-operation position, whereby HF current supply to the electrode (16) associated with the feed line is disconnected,
having an endoscope shaft (1), the distal end piece of which forms the other electrode carrier (6), at which the substantially annular first electrode (10) is placed at the end face, and an electrode cap (8) including a pin (14) supported to be axially movable in the distal end piece (6) of the endoscope shaft (1), wherein the electrode cap (8) supports a second, substantially annular electrode (16) interacting with the first electrode (10) on a side facing the end piece (6), the electrode cap (8) consisting of the rim-shaped electrode carrier (12) which is centrically supported on the now tubular pin (14) in a hinge-like manner so as to be swiveled into an operating position substantially in parallel to the end face of the distal shaft end piece (6) and into an out-of-function position at an angle of more than 0° with respect to the end face of the distal shaft end piece (6), wherein the safety switch mechanism (26, 28, 34) is mechanically coupled to the pin (14) such that at a position of the rim-shaped electrode carrier (12) outside of its operating position, the at least one feed line to the electrode (16) held by the rim-shaped electrode carrier (12) is disconnected,
wherein the rim-shaped electrode carrier (12) is pivoted relative to the pin (14) by means of an actuating rod (28) supported to be axially movable in the pin (14), the actuating rod (28) being coupled to the rim-shaped electrode carrier (12) via a joint lever (30) so as to transfer an axial movement of the actuating rod (28) along the pin (14) into a pivoting movement of the rim-shaped electrode carrier (12) about the pin (14),
and wherein the actuating rod (28) is electrically connected to the electrode (16) held by the rim-shaped electrode carrier (12) and thus constitutes a portion of the HF feed line, wherein at the tubular pin (14) a contact (26) is arranged which at a predetermined axial position of the actuating rod (28) relative to the pin (14), in which the rim-shaped electrode carrier (12) is unfolded in the operating position, enters into electric contact with the actuating rod (28) or a contact provided on the latter.

2. The medical instrument according to claim 1, **characterized in that** it is an end-to-end anastomosis HF instrument preferably of single-use design.

## Revendications

1. Instrument médical de type bipolaire avec au moins deux électrodes (10, 16) maintenues par des supports d'électrode (6, 12) mobiles l'un par rapport à l'autre, pouvant être soumises au courant HF par le biais respectivement d'une ligne d'amenée, **caractérisé par** un mécanisme disjoncteur (26, 28, 34) disposé dans la zone au moins de l'un des supports d'électrode (6, 12) et intercalé dans au moins l'une des lignes d'amenée, qui est mis dans une position d'arrêt ou de coupure seulement lors du transfert d'un premier support d'électrode (12) en forme de couronne en position de non-fonctionnement, moyennant quoi un apport de courant HF à l'électrode (16) associée à la ligne d'amenée est interrompu, avec une tige d'endoscope (1) dont l'embout distal forme l'autre support d'électrode (6) au niveau duquel la première électrode (10) sensiblement de forme circulaire est placée du côté frontal, et un capuchon d'électrode (8) avec un bâtonnet (14), qui est monté de manière à coulisser axialement dans l'embout (6) distal de la tige d'endoscope (1), dans lequel le capuchon d'électrode (8) supporte, au niveau d'une face tournée vers l'embout (6), une deuxième électrode (16) sensiblement de forme circulaire, qui coopère avec la première électrode (10), dans lequel le capuchon d'électrode (8) est constitué d'un support d'électrode (12) en forme de couronne qui est monté au centre à la manière d'une charnière sur le bâtonnet (14) désormais de forme tubulaire pour être pivoté dans une position fonctionnelle sensiblement parallèle à la face frontale de l'embout de tige (6) distal et une position de non-fonctionnement à un angle supérieur à 0° par rapport à la face frontale de l'embout de tige (6) distal, dans lequel le mécanisme disjoncteur (26, 28, 34) est couplé mécaniquement au bâtonnet (14) de sorte que, lors d'une position du support d'électrode (12) en forme de couronne en dehors de sa position fonctionnelle, l'au moins une ligne d'amenée à l'électrode (16) maintenue par le support d'électrode (12) en forme de couronne est interrompue, dans lequel le pivotement du support d'électrode (12) en forme de couronne par rapport au bâtonnet (14) est effectué au moyen d'un tige d'actionnement (28) montée mobile axialement dans le bâtonnet (14) qui est couplée au support d'électrode (12) en forme de couronne par le biais d'un levier d'articulation (30) pour transposer un mouvement axial de la tige d'actionnement (28) le long du bâtonnet (14) en un mouvement de pivotement du support d'électrode (12) en forme de couronne autour du bâtonnet (14) et dans lequel la tige d'actionnement (28) est reliée électriquement à l'électrode (16) maintenue par le support d'électrode (12) en forme de couronne et constitue ainsi une partie de la ligne d'amenée HF, dans lequel un contact tactile (26) est disposé au niveau du bâtonnet (14) de forme tubulaire, formant une fermeture de contact électrique avec la tige d'actionnement (28) ou un contact prévu au niveau de celle-ci lorsque la tige d'actionnement (28) se trouve dans une position axiale prédéfinie par rapport au bâtonnet (14) où le support d'électrode (12) en forme de couronne est extrait en position fonctionnelle.

2. Instrument médical selon la revendication 1, **caractérisé en ce que** c'est un instrument HF d'anastomose bout à bout, de préférence de type à usage unique.
